# EUROPEAN PATENT APPLICATION

(11) **EP 1 820 451 A1**
(43) Date of publication of application: **22.08.2007**
(21) Application number: 06003115.0
(22) Date of filing: 16.02.2006
(51) Int. Cl.: A61B 6/03, A61B 6/14

(54) **Tomographic x-ray apparatus and method**

(71) Applicant: Orangedental GmbH & Co. KG, 88400 Biberach (DE)
(72) Inventor: The designation of the inventor has not yet been filed
(74) Representative: Vossius, Volker

(57) **Abstract**

An apparatus for obtaining images of sectional planes in a region of interest of an object under examination is provided, wherein the apparatus comprises a radiation source for irradiating the object with penetrating electromagnetic waves, a detector diametrically opposed to the radiation source across the object for detecting electromagnetic waves transmitted through the object, scanning means for moving the radiation source and/or the detector for scanning action, and localization means for determining the position of the radiation source and/or the detector. Preferably the apparatus further comprises computing means configured for computing a three dimensional image of the object under examination based on the position corrected images of the object determined in at least two scan positions.

## Description

### TECHNICAL FIELD OF THE INVENTION

The invention relates to an apparatus and a method for obtaining images of sectional planes in a region of interest of an object under examination and for reconstructing a three dimensional image thereof. In particular, the invention relates to a tomographic x-ray apparatus for producing three dimensional images which are in particular useful in the medical field.

### BACKGROUND OF THE INVENTION

A panoramic x-ray image of the dental region and especially the dental arch of a patient (Orthopantomogram; OPG) can be obtained by means of a panoramic x-ray apparatus and allows the dentist or a orthodontist to study this region in great detail. The operating principle of most panoramic x-ray apparatus, an example of which is disclosed, e.g. in US 5,511,106, involves a turning of the x-ray source about the head of a patient, in such a manner that the dental arch will be photographed as a flat picture on a moving film or stored on some digital detection means. Thus, the basic components of a panoramic x-ray apparatus are a x-ray source, a detector, and means for moving the x-ray source and/or the detector in a suitable fashion around the dental region of the patient. During the whole scan or motion, the x-ray source is "on" and the radiation is continuously received by the detector, i.e. only a single image is produced. The skilled person will appreciate, that in order to produce good panoramic x-ray images the requirements with respect to the positional accuracy of the x-ray source and the detector are not too severe as long as the x-ray source and/or the detector are moved from their start position along their predetermined path to their end position in a continuous motion. Due to this insensitivity of a panoramic x-ray apparatus with respect to moderate displacements, e.g. oscillations, of the x-ray source and the detector relative to their nominal positions these apparatus can be relatively light weight and inexpensive.

The dentist or orthodontist may gain different or further insights by using a more sophisticated device, a so called tomographic x-ray apparatus, which allows the three-dimensional imaging of the dental region and especially the dental arch of a patient, an example of which is disclosed, e.g. in the published US application US 2002/0154728 A1. As in the case of a panoramic x-ray apparatus these devices basically consist of a x-ray source, a detector, scanning means for moving the x-ray source and the detector synchronously in a circular motion around the dental region of the patient as well as appropriate computing means to process the acquired data. Often the x-ray source and the detector are kept in a fixed spatial relationship by being attached to a solid C-shaped arch at the opposite ends thereof. Tomographic x-ray apparatus differ from panoramic x-ray apparatus essentially in that not only a single image, but a plurality of sectional images are produced which are processed by the computing means to reconstruct a three dimensional representation of the object under study. In other words, after exposing the object under study with a pulse of x-rays from the x-ray source and receiving this pulse of x-rays at the detector in a first scan position, the detector is read out and the x-ray source and the detector are moved to a second scan position and the x-ray source emits a further radiation pulse and so forth. However, these different steps can be performed so fast that the radiation source and the detector essentially move in a quasi-continuous fashion.

On the basis of the data provided by the sectional images obtained in a way as described above the computing means reconstruct a three dimensional representation of the object under study. To this end, for each scan position a position of the x-ray source and the detector has to be assumed, which is defined herein as the nominal position of the x-ray source and the detector. Generally this nominal position will be assumed to correspond to the path of the x-ray source and the detector around the object under study. Due to the increased complexity of producing a three dimensional representation out of a plurality of sectional images taken at different angles with respect to the object under study these tomographic x-ray apparatus, therefore, require a much higher positional accuracy of the x-ray source and the detector than panoramic x-ray apparatus in order to produce reliable results. In particular, the x-ray source and the detector should be exactly at the positions at which on the basis of the mechanical setup they are assumed to be for the reconstruction of the three dimensional representation. Accordingly, the x-ray source and the detector should not be allowed to oscillate about their nominal positions, i.e. their positions used for the reconstruction of the three dimensional representation. For these reasons conventional tomographic x-ray apparatus are extremely heavy and bulky, such that the x-ray source and the detector due to the mechanical setup are moved in a very precise way about the object to be imaged without virtually any oscillations about their nominal positions.

The object of the present invention is to provide a tomographic x-ray apparatus which is lighter and thus less expensive than conventional tomographic x-ray apparatus.

### SUMMARY OF THE INVENTION

The above object is achieved by an apparatus for obtaining images of sectional planes in a region of interest of an object under examination, wherein the apparatus comprises a radiation source for irradiating said object with penetrating electromagnetic waves; a detector diametrically opposed to the radiation source across the object for detecting electromagnetic waves transmitted through the object; scanning means for moving the radiation source and/or the detector from a first scan position to a second scan position; and localization means for determining the position of the radiation source and/or the detector.

Preferably, the apparatus further comprises computing means configured for computing a position corrected image of the object on the basis of the position of the radiation source and/or the detector.

Alternatively, the apparatus further comprises computing means configured for determining the displacement of the radiation source and/or said detector relative to their nominal positions and for computing a position corrected image on the basis of the displacement of the radiation source and/or the detector relative to their nominal positions.

According to a further preferred embodiment of this aspect of the invention the computing means are further configured for computing a three dimensional image of the object under examination based on the position corrected images of the object determined in at least two scan positions.

Alternatively, the apparatus further comprises computing means configured for computing a three dimensional image of the object under examination based on the position of the radiation source and/or the detector in at least two scan positions.

Preferably, the localization means comprise at least one camera. According to a still further preferred embodiment of this aspect of the invention the localization means further comprise at least one marker attached to the radiation source and/or the detector and/or the object.

Alternatively, the localization means comprise at least one laser distance sensor.

According to a still further preferred embodiment of this aspect of the invention the detector is an area detector.

Optionally, the scanning means move the radiation source and the detector synchronously in a circular motion.

According to a still further preferred embodiment of this aspect of the invention the localization means, preferably at least one camera or at least one laser distance sensor, is mounted on a fixed support decoupled from the scanning means.

Optionally, the radiation source and the detector are mounted on the respective ends of a solid C-shaped arch, such that the distance between the radiation source and the detector remains fixed.

Preferably, the localization means are further configured to determine the position of the object under examination.

According to a still further preferred embodiment of this aspect of the invention the electromagnetic waves emitted by the radiation source are x-rays or IR radiation.

According to a second aspect of the invention a method for obtaining images of sectional planes in a region of interest of an object under examination is provided. The method comprises the steps of: a) in a first scan position, irradiating the object with electromagnetic waves from a radiation source; b) detecting, essentially simultaneously, the electromagnetic waves transmitted through the object by means of a detector; and c) determining, essentially simultaneously, the position of the radiation source and/or the detector.

Preferably, the method comprises the further step of: d) computing a position corrected image of the object on the basis of the position of the radiation source and/or the detector. Optionally, the position corrected image is computed on the basis of the displacement of the radiation source and/or the detector relative to their nominal positions.

According to a still further preferred embodiment of the second aspect of the invention the method comprises the further step of: e) moving the radiation source and/or the detector to a second scan position and repeating above steps a) to d) for the second scan position.

According to a still further preferred embodiment of this second aspect of the invention the method comprises the further step of: f) reconstructing a three dimensional image of the object under examination based on the position corrected images of said object determined in at least two scan positions.

Alternatively, the method comprises the further step of: d') moving the radiation source and/or the detector to a second scan position and repeating above steps a) to c) for the second scan position and e') reconstructing a three dimensional image of the object under examination on the basis of the position of the radiation source and/or the detector in at least two scan positions.

Optionally, the radiation source and/or the detector are continuously moved and the radiation source emits radiation pulses at certain time intervals.

### BRIEF DESCRIPTION OF THE DRAWINGS

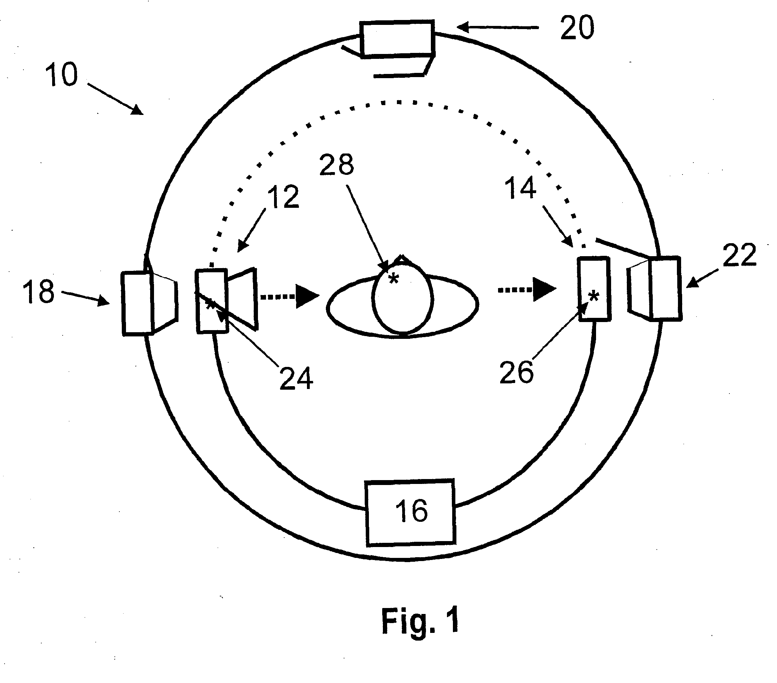

FIGURE 1 shows schematically a preferred embodiment of the tomographic x-ray apparatus according to the present invention.

### DETAILED DESCRIPTION OF PREFERRED EMBODIMENTS

A preferred embodiment of the x-ray tomographic apparatus for examining an object of interest and especially the dental region of a patient according to the present invention is shown in Figure 1. The x-ray tomographic apparatus 10 comprises a radiation source 12, e.g. a x-ray tube, and a diametrically opposed detector 14, e.g. a planar CCD chip having a plurality of pixels arranged in an array for detecting the electromagnetic wave transmitted trough the patient. The radiation source 12 and the detector 14 are preferably maintained and moved in a fixed spatial relationship with respect to each other by a C-shaped arch to which they are connected. This C-shaped arch can be moved via scanning means 16 in such a way that the radiation source 12 and the detector 14 move in a synchronous fashion along a notional circle (partially illustrated by the dotted line and partially coinciding with the C-shaped arch in figure 1), wherein the origin of this notional circle essentially coincides with the position of the patient.

In a first scan position, as shown in figure 1, the radiation source 12 emits a radiation pulse, e.g. x-rays, (illustrated by the dashed arrow in figure 1) which penetrates through the object of interest and reaches the detector 14. The radiation source 12 and the detector 14 are connected to control means (not shown) which control and time the emission of x-ray pulses by the radiation source 12, the read-out from the detector 14 as well as the actuation of the scanning means 16 in order to move the radiation source 12 and the detector 14, as will be described in more detail further below. As soon as the control means have read out the image in a first scan position and have transferred this data to the computing means, the scanning means 16 move the radiation source 12 and the detector 14 by means of the C-shaped arch into a second scan position along the notional circle. At this second scan position the radiation source 12 again emits a pulse of radiation, e.g. x-rays, which penetrates through the object of interest and reaches the detector 14 in this second scan position, the output of which also is transferred to the computing means as the sectional image in the second scan position.

Preferably, this process continues until a certain scan region comprising at least two scan positions has been covered and a corresponding number of sectional images has been produced. The person skilled in the art will appreciate that the above described process can be performed very fast, such that the radiation source and the detector essentially perform a quasi-continuous motion, wherein a radiation pulse is emitted at certain time intervals.

The tomographic x-ray apparatus according to the present invention, furthermore, comprises localization means for determining the exact position of the radiation source and/or the detector and thus any deviations from their nominal positions in every scan position. As already described above, the nominal position of these components is defined as the position the radiation source and the detector should be at due to the mechanical setup of the apparatus, which generally corresponds to the position used for the reconstruction of a three dimensional image. The localization means according to the present invention preferably comprise at least one camera and at least one marker attached to the radiation source and/or the detector. Furthermore, at least one marker can be provided on the object under study, so that also its position can be localized by the localization means, preferably a camera.

In the exemplary embodiment of Figure 1 the apparatus 10 comprises three cameras 18, 20, 22 which are mounted on a support, e.g. a solid ring, which in turn can be attached e.g. to the ceiling of the room the tomographic x-ray apparatus is located in. The person skilled in the art will appreciate that these cameras can be installed in a different way, as long as they are maintained in a fixed positional relationship with respect to each other. The cameras 18, 20, 22 are configured and arranged to determine the position of the radiation source 12, the detector 14 and the object under study with a very high precision. As most of the displacements of the radiation source 12 and the detector 14 with respect to their nominal positions occur in directions perpendicular to the line connecting the radiation source 12 and the detector 14, the cameras should be configured and arranged, such that they are especially sensitive with respect to such motions.

If the radiation source 12 and the detector 14 are connected by means of a solid C-shaped arch, as shown in figure 1, then in principle it is sufficient to determine only the position of one of these components. On the basis of this position and the fixed spatial relationship due to the C-shaped arm, the position of the other component easily can be determined. However, as shown in figure 1, it is also possible that the positions of the radiation source 12, the detector 14 and the object under study are determined separately and stored for each scan position and thus for each sectional image of the object under study.

After a full scan of the tomographic x-ray apparatus according to the present invention the computing means are provided with a sectional image of the object under study and an exact position of the radiation source and the detector for each scan position. On the basis of this data the computing means can compute the displacement of the radiation source and the detector relative to their nominal position and a position corrected sectional image of said object under study. Such a position corrected image will be computed for each scan position and will be used for the three dimensional reconstruction of the object under study.

The markers, which represent the target points of the localization means, preferably at least one camera, preferably have some visual structure such that not only the position of the marker within a two dimensional field of view but also its angular orientation with respect to the line of sight between the localization means, preferably the camera and the marker can be determined. A preferred marker has the shape of a small circle, wherein one pair of diametrically opposed quadrants of the circle is black and the other pair of quadrants is white. The person skilled in the art will appreciate, that a further improvement of the determination of the position of the radiation source and/or the detector can be obtained by using more than one marker on each component. Furthermore, the person skilled in the art will appreciate, that instead of markers also characteristic features of the radiation source, the detector or the object itself could be used as target points for the determination of the exact position thereof.

The person skilled in the art will appreciate that instead of cameras also laser distance sensors or any other appropriate means could be used as localization means. Available laser distance sensors allow to determine the distance of an object with a precision of about 10 microns within a range of up to 50 cm. It is a matter of routine work for the skilled person in the art to determine depending on the size of the whole tomographic x-ray apparatus how many of such conventional laser distance sensors would be required to provide the necessary information about the exact positions of the radiation source and/or the detector in every scan position. As the specifics of laser distance sensors are well known to the person skilled in the art the details thereof will not be described in greater detail.

While the invention has been described with respect to preferred embodiments, those skilled in the art will readily appreciate that various changes and/or modifications can be made to the invention without departing from the scope of the invention as defined by the appended claims.

## Claims

1. An apparatus (10) for obtaining images of sectional planes in a region of interest of an object under examination, said apparatus (10) comprising:
a radiation source (12) for irradiating said object with penetrating electromagnetic waves;
a detector (14) diametrically opposed to said radiation source (12) across said object for detecting electromagnetic waves transmitted through said object;
scanning means (16) for moving said radiation source (12) and/or said detector (14) from a first scan position to a second scan position; and
localization means for determining the position of said radiation source (12) and/or said detector (14).

2. Apparatus of claim 1, wherein the apparatus further comprises
computing means configured for computing a position corrected image of said object on the basis of the position of the radiation source (12) and/or the detector (14).

3. Apparatus of claim 1 or 2, wherein the apparatus further comprises
computing means configured for determining the displacement of said radiation source (12) and/or said detector (14) relative to their nominal positions and for computing a position corrected image on the basis of the displacement of the radiation source and/or the detector relative to their nominal positions.

4. Apparatus of any of claims 1 to 3, wherein the computing means are further configured for computing a three dimensional image of the object under examination based on the position corrected images of the object determined in at least two scan positions.

5. Apparatus of claim 1, wherein the apparatus further comprises
computing means configured for computing a three dimensional image of the object under examination on the basis of the position of the radiation source (12) and/or the detector (14) in at least two scan positions.

6. Apparatus of any one of the preceding claims, wherein the localization means comprise at least one camera (18, 20, 22).

7. Apparatus of claim 6, wherein the localization means further comprise at least one marker (24, 26, 28) attached to the radiation source (12) and/or the detector (14) and/or the object.

8. Apparatus of claims 1 to 5, wherein the localization means comprise at least one laser distance sensor.

9. Apparatus of any one of the preceding claims, wherein the detector (14) is an area detector.

10. Apparatus of any one of the preceding claims, wherein the scanning means (16) move said radiation source (12) and said detector (14) synchronously in a circular motion.

11. Apparatus of any one of claims 6 and 7, wherein said at least one camera (18, 20, 22) is mounted on a fixed support decoupled from the scanning means (16).

12. Apparatus of any one of the preceding claims, wherein the radiation source (12) and the detector (14) are mounted on the respective ends of a solid C-shaped arch, such that the distance between the radiation source (12) and the detector (14) remains fixed.

13. Apparatus of any one of the preceding claims, wherein said localization means are further configured to determine the position of the object under examination.

14. Apparatus of any one of the preceding claims, wherein said electromagnetic waves are x-rays or IR radiation.

15. A method for obtaining images of sectional planes in a region of interest of an object under examination, said method comprising the following steps:
a) in a first scan position, irradiating said object with electromagnetic waves from a radiation source (12);
b) detecting, essentially simultaneously, the electromagnetic waves transmitted through said object by means of a detector (14); and
c) determining, essentially simultaneously, the position of said radiation source (12) and/or said detector (14).

16. Method of claim 15, wherein the method comprises the further step of:
d) computing a position corrected image of the object on the basis of the position of the radiation source (12) and the detector (14).

17. Method of claim 16, wherein the position corrected image is computed on the basis of the displacement of the radiation source (12) and/or the detector (14) relative to their nominal positions.

18. Method of claims 16 or 17, wherein the method comprises the further step of:
e) moving said radiation source (12) and/or said detector (14) to a second scan position and repeating steps a) to d) for the second scan position.

19. Method of claim 18, wherein the method comprises the further step of:
f) reconstructing a three dimensional image of the object under examination based on the position corrected images of said object determined in at least two scan positions.

20. Method of claim 15, wherein the method comprises the further steps of:
d') moving said radiation source (12) and/or said detector (14) to a second scan position and repeating steps a) to c) for the second scan position; and
e') reconstructing a three dimensional image of the object under examination on the basis of the position of the radiation source (12) and/or the detector (14) in at least two scan positions.

21. Method of any one of claims 18 to 20, wherein the radiation source (12) and/or the detector (14) are continuously moved and the radiation source (12) emits radiation pulses at certain time intervals.
